(19) Europäisches Patentamt
European Patent Office
Office européen des brevets

(11) Publication number: 0 420 766 B1

## (12) EUROPEAN PATENT SPECIFICATION

(45) Date of publication of patent specification: **01.03.95**  (51) Int. Cl.6: **A61M 1/38**

(21) Application number: **90402697.8**

(22) Date of filing: **28.09.90**

(54) **Blood cleaning apparatus and method for cleaning blood therewith.**

(30) Priority: **29.09.89 JP 254033/89**

(43) Date of publication of application:
**03.04.91 Bulletin 91/14**

(45) Publication of the grant of the patent:
**01.03.95 Bulletin 95/09**

(84) Designated Contracting States:
**BE DE FR GB IT NL SE**

(56) References cited:
**EP-A- 0 173 631**

**BIOLOGICAL ABSTRACTS, vol. 64, abstract no. 36983, Philadelphia, PA, US; S.MOORJANI et al.: "Extracorporeal removal of plasma lipo proteins by affinitybinding to heparin agarose", & CLINICA CHIMICA ACTA 77(1),1977, pages 21-30**

(73) Proprietor: **TERUMO KABUSHIKI KAISHA No. 44-1, Hatagaya 2-chome, Shibuya-ku Tokyo 151 (JP)**

(72) Inventor: **Yura, Yoshifumi, c/o Terumo K. K. 1500, Inokuchi, Nakai-cho Ashigarakami-gun, Kanagawa-ken (JP)**
Inventor: **Nagoya, Masako, c/o Terumo K. K. 1500, Inokuchi, Nakai-cho Ashigarakami-gun, Kanagawa-ken (JP)**
Inventor: **Okawara, Junichi, c/o Terumo K. K. 1500, Inokuchi, Nakai-cho Ashigarakami-gun, Kanagawa-ken (JP)**
Inventor: **Yamamoto, Yuichi, c/o Terumo K. K. 1500, Inokuchi, Nakai-cho Ashigarakami-gun, Kanagawa-ken (JP)**

(74) Representative: **Gillard, Marie-Louise et al Cabinet Beau de Loménie 158, rue de l'Université F-75340 Paris Cédex 07 (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

Field of the Invention:

This invention relates to a blood cleaning apparatus and a method for cleaning blood therewith. More particularly, it relates to an electrical blood cleaning apparatus for selective adsorption of low density lipoprotein in blood plasma and a method for cleaning blood therewith.

Description of the Prior Art:

EP-A-0 173 631 describes an apparatus for continuously fractionating by electrophoresis solutions containing proteins and more particularly human plasma.

SU-A-1 074 493 describes an haemosorption method according to which blood is let through a sorbent under the action of an electrical field. Said sorbent is placed between two vibrating electrodes. Said electrodes and sorbent are not in contact. The method may be used to remove bilirubin and urea from the blood.

The cholesterol which is held to constitute a pathogenic substance inducing ischemic cardiac diseases and various vascular disorders as well as chemical-resistant nephrosis originating in the simple glomerulosclerosis is sparingly soluble in water and, therefore, maintains its existence in blood while forming lipoprotein particles in conjunction with triglycerides, phospholipids, and apoproteins. These lipoproteins are divided by density into very low density lipoprotein (VLDL), low density lipoprotein (LDL), and high density lipoprotein (HDL), for example. Among other lipoproteins mentioned above, LDL has a particularly high cholesterol content. In normal persons, the LDL is maintained at a stationary level through the metabolism of blood plasma lipoprotein in the liver. In the cases of familial hypercholesterolemia attended by hereditary absence of LDL receptor from the liver, an abnormal increase of LDL induces juvenile ischemic cardiac diseases. Particularly in homozygotes and serious heterozygotes of familial hypercholesterolemia, the pharmacotherapy is incapable of lowering the cholesterol concentration to a level low enough to prevent arterioscelerosis from advancing. A sufficient decrease of the cholesterol concentration dictates an extracorporeal blood cleaning treatment.

As one way of effecting the blood cleaning for the removal of LDL, the blood plasma exchange therapy resorting to continuous centrifugal separation and use of frozen blood plasma has been tried by Thompson et al. and consequently demonstrated to bring about a high clinical effect of LDL reduction (Thompson, G. R., et al.: Plasma exchange in the management of homozygous familial hypercholesterolamia, Lancet 1, 1208 (1975)). This method, however, necessitates a complicate and expensive apparatus and entails the disadvantage that the treatment of centrifugal separation result in thorough discard of other useful components present in the blood plasma under treatment.

From the standpoint of allowing selective removal of LDL with higher accuracy, various methods have been proposed which may be broadly classified under the three methods, i.e. the methods resorting to filtration through a membrane, the methods resorting to fractional precipitation, and the methods resorting to selective adsorption. These methods, however, have many problems yet to be solved. The double filtration plasmapheresis, one of the membrane filtration methods, for example, effects fractionation of LDL and HDL by molecular size only incompletely and consequently suffers admission of free cholesterol and loss of various forms of useful blood plasma proteins and HDL which are presumed to serve as suppressive factors for hypercholesterolemia. The heparin-calcium precipitation method, one of the fractional precipitation methods, is selective relatively to LDL and yet unsatisfactory in terms of the amount of LDL to be removed and, therewith, obliged to use calcium in a concentration approximately 10 times of the physiological concentration. The selective adsorption method is not fully reliable as to accuracy of removal and capacity for removal. When this method is performed by the use of an adsorbent having immobilized thereon an anti-LDL antibody and an acidic mucopolysaccharide as affinity ligands for the purpose of enhancing selectivity of the treatment, it inevitably poses problems in terms of stability in the light of liberation of ligands and in terms of economy.

An object of this invention, therefore, is to provide a novel blood cleaning apparatus and a method for cleaning blood therewith.

Another object of this invention is to provide a blood cleaning apparatus for selective and highly accurate removal by adsorption of LDL from blood and a method for cleaning blood therewith.

Yet another object of this invention is to provide a blood cleaning apparatus endowed with an ability to effect selective removal by adsorption of LDL at no sacrifice of useful substances such as HDL and albumin-containing blood plasma proteins and allow removal of LDL with efficiency high enough to compare favorably with the efficiency attainable by the blood plasma exchange therapy and a method for cleaning blood therewith.

A further object of this invention is to provide a blood cleaning apparatus excelling in stability evinced by resistance to the conditions of treatment in an autoclave and absence of liberation of ligands and in economy and operational simplicity and a method for cleaning blood therewith.

## SUMMARY OF THE INVENTION

The objects described above are accomplished by a blood cleaning apparatus for selective removal by adsorption of an unwanted substance from blood, which apparatus being provided with an electrically polarized solid surface as an adsorbent carrier.

The apparatus of the invention is more precisely characterized in that it is provided with a solid surface purely and directly polarized by the use of electrical devices and electrical circuits ; said polarized solid surface forming an electrode and acting as an adsorbent carrier for selective removal by adsorption of an unwanted substance possessing a molecular structure of high electrostatic anisotropy in the blood.

This invention also discloses a blood cleaning apparatus, wherein the electrically polarized solid surface selectively adsorbs as LDL as an unwanted substance in blood.

The objects described above are further accomplished by a method for cleaning blood, which method is characterized by causing blood requiring treatment to contact a solid surface purely and directly polarized by the use of electrical devices and electrical circuits and thus forming an electrode thereby effecting selective removal of an unwanted substance contained in the blood and possessing a molecular structure of highly electrostatic anisotropy by adsorption of said unwanted substance on said solid surface by virtue of an electrostatic interaction.

This invention also discloses a method for cleaning blood, wherein LDL is selectively removed by adsorption from blood plasma.

This invention, as described above, is directed to a blood cleaning apparatus characterized by being provided with an electrically polarized solid surface as an adsorbent carrier and to a method for cleaning blood characterized by causing blood subjected to treatment to contact an electrically polarized solid surface thereby utilizing the resultant electrostatic interaction for effecting selective removal by adsorption of an unwanted substance possessing a molecular structure of high electrostatic anisotropy in blood. Thus, the present invention allows such an unwanted substance as LDL possessing a molecular structure of high electrostatic anisotropy to be removed by highly selective adsorption due to the electrostatic interaction and, therefore, accomplishes highly selective cleaning of blood plasma. This invention, therefore, promises that when a varying extra-corporeal circulation circuit has the blood cleaning apparatus of this invention inserted therein, it can be effectively applied to the therapy for removing by adsorption the cholesterol, a presumable pathogenic substance inducing ischemic cardiac diseases and various vascular disorders as well as chemical-resistant nephrosis orifinating in the simple glomerulosclerosis, with efficiency high enough to compare favorably with the efficiency attainable by the blood plasma exchange therapy without any sacrifice of the stability of the apparatus evinced by resistance to the conditions of treatment in an autoclave or preclusion of liberation of ligands, the economy of the therapy, and the operational convenience of the apparatus. Further, since this invention utilizes as an adsorbent carrier a solid surface purely and directly polarized by the use of electrical devices and electrical circuits, the stability of performance and the principle of adsorption can be easily comprehended from the industrial point of view because this solid surface is not interfered with by other nonspecific interactions than the electrostatic interaction as compared with a solid surface having a functional group incorporated therein chemically. Moreover, since this invention allows the polarizing property and other electrical properties on the solid surface to be freely varied mechanically, a desired capacity for electrostatic interaction can be freely set on one and the same solid surface. This invention, therefore, may well be called an extensively versatile and highly useful blood cleaning technique capable of effecting mutual separation of extraneous components in blood such as, for example, high molecular substances like various blood plasma proteins possessing structures rich in electrostatic anisotropy, low molecular substances like bile acid and ion seeds, and blood cells possessing a membrane formed of such molecules without requiring any alteration in the construction of apparatus.

## BRIEF DESCRIPTION OF THE DRAWINGS

Figs. 1 to 4 are diagrams each illustrating a varying principle of application of voltage for the production of a state of polarization usable for the blood cleaning apparatus of the present invention,

Figs. 5 is a typical diagram illustrating a construction of the blood cleaning apparatus as one embodiment of the present invention, and

Fig. 6 is a typical diagram illustrating a construction of the extra-corporeal circulation circuit incorporating therein the blood cleaning apparatus as one embodiment of this invention.

EXPALNATION OF PREFERRED EMBODIMENT

The blood cleaning apparatus of this invention utilizes an electrically polarized solid surface as an adsorbent carrier and, therefore, allows substances of molecular structures of high electrostatic anisotropy contained in blood to be highly selectively removed by adsorption due to an electrostatic interaction.

Now, the mechanism which underlies this selective adsorption will be described in detail below with reference to embodiments of the invention. The LDL forms a specifically sedimenting complex with an acidic mucopolysaccharide, a polyanion, as heretofore utilized in the fractional precipitation method. From this fact, it is inferred that the LDL possesses a molecular structure of high electrostatic anisotropy readily interacting with a polyanion. When blood is brought into contact with a solid surface negatively polarized purely and directly as by the use of electrical devices and electrical circuits, therefore, the LDL contained in the blood is presumably adsorbed highly selectively on the solid surface owing to the same electrostatic interaction as with the acidic mucopolysaccharide. Further, the present invention utilizes as an adsorbent carrier a solid surface purely and directly polarized by the use of electrical devices and electrical circuits. The stability of performance of the apparatus and the principle of adsorption, therefore, can be easily comprehended because the adsorbent carrier is not interfered with by other nonspecific interactions than the electrostatic interaction as compared with a solid surface having an acidic functional group chemically incorporated therein (such as, for example, a carrier having an acidic mucopolysaccharide immobilized thereon).

Further, in this invention, since the polarizing property and other similar electrical properties on the solid surface can be freely varied mechanically, the electrostatic interacting power can be freely set on one and the same solid surface. The present invention, therefore, allows mutual separation of extraneous components in blood such as, for example, high molecular substances like various blood plasma proteins possessing structures of electrostatic anisotropy, low molecular substances like bile acid and ion seeds, and blood cells possessing a membrane formed of such molecules without requiring any alteration in apparatus.

Now, the present invention will be described more specifically below with reference to embodiments thereof.

The blood cleaning apparatus of this invention is characterized by being provided with an electrically polarized solid surface as an adsorbent carrier. The term "polarization" as used in the present invention refers to a change in the electrical polarity formed on the surface of an electrode or on a non-conductive solid surface with an electric field generated by application of voltage between two electrodes.

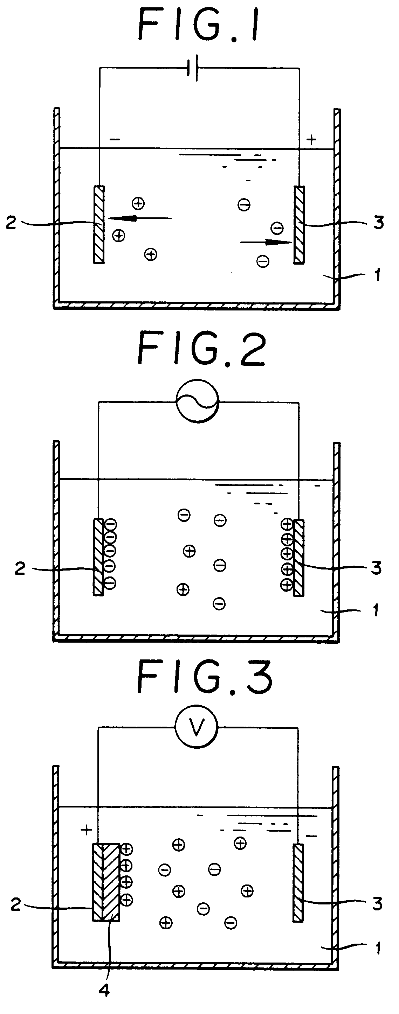

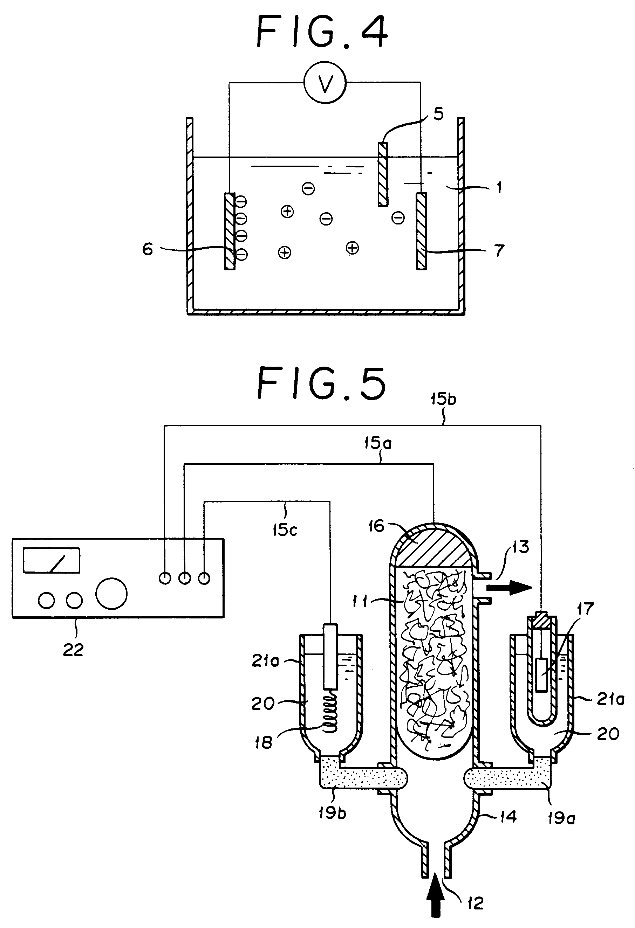

The application of voltage may be effected by any method available therefor at all on the sole condition that a DC voltage or an AC voltage can be applied between two electrodes in an electrolyte and the electric charge on the solid surface inclusive of the electrodes themselves can be controlled by some means or other. A method which comprises directly passing an electric current between electrodes 2 and 3 in a DC electric field in an electrolyte 1 thereby producing a physical force or migration as illustrated in Fig. 1, a method which comprises applying an AC electric field of a frequency exceeding a prescribed level between the electrodes 2 and 3 in an electrolyte 1 thereby polarizing the phase boundaries of the electrodes 2 and 3 themselves proportionately to the material of the electrodes, the kind of the electrolyte, the intensity of the electric field, and the magnitude of the frequency as illustrated in Fig. 2, a method which comprises interposing an insulator 4 (inclusive of a vacuum space) between the electrodes 2 and 3 thereby producing a surface polarized proportionate to the dielectric constant of the insulator 4 as illustrated in Fig. 3, and a method which comprises electrochemically controlling the potential between a working electrode 6 and a reference electrode 5 possessing a prescribed reference potential within a range incapable of inducing electrolysis of the electrolyte 1 by the use of the reference electrode 5 and a counter electrode 7 as illustrated in Fig. 4 are available, for example. Optionally, the electric current may be produced by means of a dynamic magnetic field. Particularly when the solid surface of the electrodes themselves or the insulator is polarized, the electrical interaction excerted on the adsorbed molecules during the course of adsorption is believed to be especially large because the change in an electrical phenomenon, namely the energy exerted on the system, is concentrated only in very close proximity to the solid boundary of the electrodes or the insulator. The electrochemical control method is used advantageously because the electrodes themselves can be utilized as an adsorbent carrier and the electric charge can be effectively set on the solid surface of the electrodes by means of a system of very low voltage and low energy.

The electrical devices and the electrical circuits which are used in this case may be identical in structure to those used in the known electrolytic cell. For the control of the electrode potential, the potentiostat which is in popular use may be employed. The connection between the reference electrode and the counter electrode may be formed with a salt bridge. A silver/silver chloride electrode or a saturated calomel electrode, for example, can be used advantageously as the reference electrode and platinum or carbon, for example, as the counter electrode. The working electrode which concurrently serves as an adsorbent carrier is preferable to be formed of a material which exhibits a high polarizing property in the electrolytic solution. The materials which are usable advantageously therefor include zinc, platinum, gold, and carbon, for example. The semiconductor such as of indium oxide doped with tin oxide can be advantageously used as the working electrode. The working electrode is not discriminated at all on account of shape. In order for the working electrode to acquire a large surface area for adsorption, a porous web, a bundle of microfilaments, or a non-woven or woven fabric of microfilaments may be used to advantage. The component parts described above are only required to complete the structure of an electrolytic by being stowed in a proper housing.

The materials which are usable advantageously for the construction of the housing include synthetic resins such as polyethylene, polypropylene, polycarbonate, polystyrene, and polymethyl methacrylate, and glass. Among other materials mentioned above, polypropylene and polycarbonate prove to be particularly desirable because they tolerate the impact of hydrothermal sterilization and permit easy handling.

Fig. 5 is a cross section typically illustrating the structure of one example of the blood cleaning apparatus of the present invention. As illustrated in Fig. 5, a working electrode 11 formed of a bundle of graphite fibers is packed as an adsorbent carrier with a prescribed bulk density in the range of 0.5 to 1.5 $g/cm^3$, for example, in a column 14 provided with a blood inlet 12 and a blood outlet 13. It has a point of contact with a conductor 15a in the blood outlet 13 side end part of the column. It is insulated and immobilized inside the column 14 with an insulating adhesive agent 16 made of epoxy resin, urethane resin, or low-melting glass, for example. A reference electrode 17 formed of a saturated calomel electrode and a counter electrode 18 formed of a platinum wire establish contact with the column 14 packed with the working electrode 11 through the salt bridges 19a and 19b filled with agar gel and are fixed respectively in separate baths 21a and 21b which are filled with an electrolyte 20 formed of saturated sodium chloride solution. The conductors 15a, 15b, and 15c respectively led out of the electrodes 11 and 17, and 18 are connected to a potentiostat 22. It is with this potentiostat 22 that the electrode potential of the working electrode 11 formed of a bundle of graphite fibers is regulated.

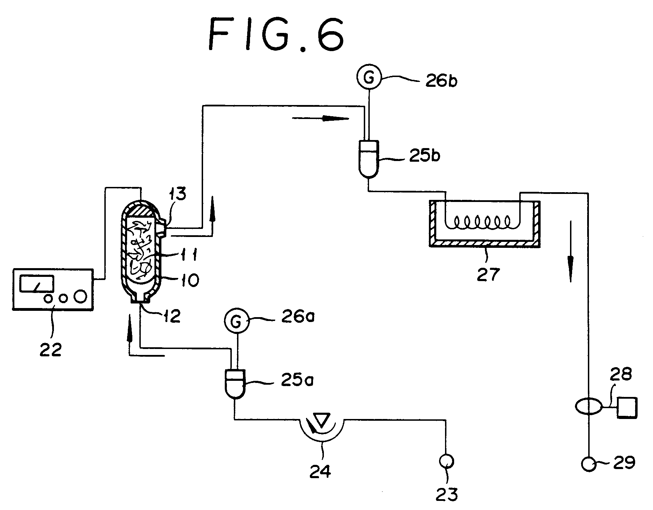

The blood cleaning apparatus constructed as described above is used advantageously for selective removal from blood by adsorption of unwanted substances represented by LDL and possessed of a molecular structure of high electrostatic anisotropy. When the blood from a patient of hypercholesterolemia is subjected to extracorporeal circulation for the purpose of purging the blood of LDL, for example, the incorporation in the blood path of the blood cleaning apparatus of the aforementioned construction as illustrated in Fig. 6 suffices to attain the desired removal of LDL. More specifically, the blood from the patient is introduced via a blood inlet 23 into the circuit, passed through a blood pump 24 and a chamber 25a, and supplied at a constant rate of flow to the blood cleaning apparatus 10. The chamber 25a is provided with a pressure gauge 26a and consequently allowed to monitor the flow resistance to the blood 10 in circulation therethrough. Inside the blood cleaning apparatus 10, the blood introduced via the blood inlet 12 contacts the bundle of graphite fibers serving as a working electrode 11 polarized at a prescribed potential in the range of -0.9 to +1.7 V (relative to the reference electrode), preferably -0.6 to 0.4 V (relative to the reference electrode) and undergoes a treatment for adsorption of LDL. The blood resulting from this treatment is led out of the blood outlet 13. The blood emanating from the blood outlet 13 is passed through a monitoring unit composed of the chamber 25b and the pressure gauge 26b and then through a constant temperature bath 27 and a bubble detecting device 28 and returned via a blood outlet 29 into the patients body. The present embodiment has been described as a one having the whole blood directly supplied to the blood cleaning apparatus 10. It is of course allowable to separate the blood from the patient preparatorily into a blood cell component and a blood plasma component as by centrifugal separation, subject the blood plasma component alone to the treatment with the blood cleaning apparatus 10, combine the cleaned blood plasma component with the blood cell component, and return the outcome of the combination to the patient's body.

The blood cleaning apparatus of this invention is hydrothermally sterilized prior to use by filling the interior of the apparatus with refined water or physiological saline solution and heating the apparatus. Now, the present invention will be described more specifically below with reference to working examples.

### Example 1

A column was produced by filling a polycarbonate pipe 11 mm inside diameter with a working electrode formed of graphite fibers 7 $\mu$m in diameter (produced by Toho Rayon Co., Ltd. and marketed under trademark designation of "Besfite") in a density of 0.9 g/cm$^3$, connecting one end of the pipe with a conductor, and immobilizing the working electrode with epoxy resin in the pipe. To this column, a reference electrode formed of a saturated calomel electrode (S.C.E.) and a counter electrode formed of a platinum electrode were connected through the medium of salt bridge (2% agar gel). The potential between the electrodes was controlled with a potentiostat (produced by Toho Giken K.K. and marketed under product code of "2000").

To the graphite fibers in the column constituting itself an electrolytic cell, under a set potential of -0.9 V vs S.C.E., 1.5 ml of hypercholesteremic blood plasma extracted from a rabbit fed with hypercholesteremic feed for three months was injected at a flow speed of 0.1 ml/min.

Thereafter, the blood plasma recovered was tested for LDL concentration ($\beta$-lipoprotein C test) by the use of a LDL measuring kit (produced by Wako Junyaku K.K.). The results are shown in Table 1.

### Example 2

An experiment was carried out by following the procedure of Example 1, except that the set potential was -0.7 V vs. S.C.E. The results are shown in Table 1.

### Example 3

An experiment was carried out by following the procedure of Example 1, except that the set potential was -0.6 V vs. S.C.E. The results are shown in Table 1.

### Example 4

An experiment was carried out by following the procedure of Example 1, except that the set potential was changed to -0.55V vs. S.C.E. besides being tested for LDL concentrations, and the recovered blood plasma was tested for HDL concentration, total protein concentration, and albumin concentration by the use of commercially available test reagents (resepctively by the HDL-cholesterol test and by the A/GB tests using a protein testing buret solution and an alubumin coloring reagent, produced invariably by Wako Junyaku K.K.). The results are shown in Table 1.

### Example 5

An experiment was carried out by following the procedure of Example 4, except that the set potential was changed to -0.5 V vs. S.C.E. The results are shown in Table 1.

### Example 6

An experiment was carried out by following the procedure of Example 4, except that the set potential was changed to -0.45 V vs S.C.E. The results are shown in Table 1.

### Example 7

An experiment was carried out by following the procedure of Example 1, except that the set potential was changed to -0.4 V vs S.C.E. The results are shown in Table 1.

### Example 8

An experiment was carried out by following the procedure of Example 1, except that the set potential was changed to -0.25 V vs S.C.E. The results are shown in Table 1.

Example 9

An experiment was carired out by following the procedure of Example 1, except that the set potential was changed to 0.15 V vs S.C.E. The results are shown in Table 1.

Example 10

An experiment was carried out by following the procedure of Example 1, except that the set potential was changed to 0.5 V vs S.C.E. The results are shown in Table 1.

Control 1

An experiment was carried out by following the procedure of Example 1, except that no potential was set for the graphite fibers filling the column. The results are shown in Table 1.

Example 11

To the graphite fibers filling the column produced in the same manner as in Example 1, under a set potential of -0.5 V vs S.C.E., 15 ml of the hypercholesteremic blood plasma from the rabbit was circulated at a flow speed of 1 ml/min. The sample blood plasmas taken after 15, 30, 60, and 120 minutes' circulation were tested for protein concentration. The results are shown in Table 2.

Table 1

| (n = 4) | LDL adsorption ratio (%) | HDL adsorption ratio (%) | Total protein adsorption ratio (%) | Albumin adsorption ratio (%) |
|---|---|---|---|---|
| Initial value* | (1673 mg/dl) | (32.2 mg/dl) | (67.2 mg/ml) | (33.4 mg/ml) |
| Control 1 | 27.4 | | | |
| Example 1 | 58.9 | | | |
| Example 2 | 60.0 | | | |
| Example 3 | 77.3 | | | |
| Example 4 | 82.1 | 1.4 | 10.7 | 8.1 |
| Example 5 | 92.7 | 3.7 | 13.7 | 8.2 |
| Example 6 | 87.0 | 2.0 | 18.4 | 8.0 |
| Example 7 | 78.5 | | | |
| Example 8 | 59.3 | | | |
| Example 9 | 48.4 | | | |
| Example 10 | 34.6 | | | |

*) Concentration of varying proteins contained in 1.5 ml of hypercholesteremic blood plasma from the rabbit prior to passage through the cleaning apparatus.
Colum capacity 3.8 cm$^3$, Packing density 0.9 g/cm$^3$

8

Table 2

| Time of circulation (min.) | LDL adsorption ratio (%) | HDL adsorption ratio (%) | Albumin adsorption ratio (%) |
|---|---|---|---|
| 0 | 0 | 0 | 0 |
| 15 | 13.4 | 4.9 | 3.6 |
| 30 | 24.5 | 1.1 | 3.8 |
| 60 | 35.3 | 2.0 | 4.5 |
| 120 | 56.8 | -4.3 | 7.7 |

Flow speed 1 ml/min, Column capacity 3.8 ml, Amount of circulation 15 ml.

It is clearly noted from the results shown in Table 1 and Table 2 that the blood cleaning apparatus of this invention effectively removed by adsorption only the LDL at virtually no sacrifice of the other blood plasma proteins which are useful components of the blood plasma.

The method of the invention using a blood cleaning apparatus provided with an electrically polarized solid surface as an adsorbent carrier has been described for the cleaning of blood, removed from a patient.

EP 0 420 766 B1

It has to be noted that said method may be carried out in other contexts.
For example blood stored in a blood-bank may be cleaned according to the method of the invention.

**Claims**

1. A blood cleaning apparatus provided with a solid surface (11) purely and directly polarized by the use of electrical devices and electrical circuits (17, 18, 15a, 15b, 15c, 22); said polarized solid surface (11) forming an electrode characterized in that said solid surface (11) acts as an adsorbent carrier for selective removal by adsorption of an unwanted substance possessing a molecular structure of high electrostatic anisotropy in the blood.

2. An apparatus according to claim 1, wherein said unwanted substance in blood to be selectively adsorbed on the electrically polarized solid surface is low density lipoprotein.

3. A method for blood cleaning, subject to a disclaimer to methods for treatment of a human or animal body by therapy, characterized by causing blood subjected to treatment to contact a solid surface (11) purely and directly polarized by the use of electrical devices and electrical circuits (17, 18, 15a, 15b, 15c, 22) and thus forming an electrode ; thereby effecting selective removal of an unwanted substance contained in the blood and possessing a molecular structure of highly electrostatic anisotropy by adsorption of said unwanted substance on said solid surface (11).

4. A method according to claim 3, wherein low density lipoprotein is selectively removed by adsorption from blood plasma.

**Patentansprüche**

1. Blutreinigungsgerät mit einer massiven oder Feststoff-Fläche (11), die rein (lediglich) und unmittelbar mittels elektrischer Vorrichtungen und elektrischer Schaltungen (17, 18, 15a, 15b, 15c, 22) polarisiert ist oder wird, wobei die polarisierte Feststoff-Fläche (11) eine Elektrode bildet, dadurch gekennzeichnet, daß die Feststoff-Fläche (11) als ein Adsorbensträger für die durch Adsorption erfolgende selektive Entfernung einer im Blut enthaltenen unerwünschten Substanz mit einer Molekularstruktur einer hohen elektrostatischen Anisotropie wirkt.

2. Gerät nach Anspruch 1, wobei die im Blut enthaltene und selektiv an die elektrisch polarisierte Feststoff-Fläche zu adsorbierende unerwünschte Substanz niedrigdichtes Lipoprotein ist.

3. Verfahren zur Blutreinigung, mit dem Ausschluß von Verfahren zur therapeutischen Behandlung eines menschlichen oder tierischen Körpers, dadurch gekennzeichnet, daß das zu behandelnde Blut mit einer massiven (solid) oder Feststoff-Fläche (11), die mittels elektrischer Vorrichtungen und elektrischer Schaltungen (17, 18, 15a, 15b, 15c, 22) rein (lediglich) und unmittelbar polarisiert ist und damit eine Elektrode bildet, in Berührung gebracht wird und dadurch die selektive Entfernung einer im Blut enthaltenen unerwünschten Substanz mit einer Molekularstruktur hoher elektrostatischer Anisotropie durch Adsorption der unerwünschten Substanz an der (die) Feststoff-Fläche (11) bewirkt wird.

4. Verfahren nach Anspruch 3, wobei niedrigdichtes Lipoprotein durch Adsorption selektiv aus Blutplasma entfernt wird.

**Revendications**

1. Appareil de purification du sang doté d'une surface solide (11) polarisée purement et directement par l'emploi de dispositifs électriques et de circuits électriques (17, 18, 15a, 15b, 15c, 22) ; ladite surface solide polarisée (11) formant une électrode, caractérisé en ce que ladite surface solide (11) joue le rôle de support adsorbant pour l'enlèvement sélectif par adsorption d'une substance indésirable possédant une structure moléculaire présentant une forte anisotropie électrostatique dans le sang.

2. Appareil selon la revendication 1, dans lequel ladite substance indésirable du sang qui doit être adsorbée sélectivement sur la surface solide polarisée électriquement est une lipoprotéine de basse densité.

10

3. Procédé de purification du sang, à l'exclusion des méthodes de traitement thérapeutique du corps humain ou animal, caractérisé par la mise en contact du sang soumis au traitement avec une surface solide (11) polarisée purement et directement par l'emploi de dispositifs électriques et de circuits électriques (17, 18, 15a, 15b, 15c, 22) et formant ainsi une électrode ; ce qui induit l'enlèvement sélectif d'une substance indésirable contenue dans le sang et possédant une structure moléculaire présentant une forte anisotropie électrostatique par adsorption de ladite substance indésirable sur ladite surface solide (11).

4. Procédé selon la revendication 3, dans lequel une lipoprotéine de basse densité est retirée sélectivement du plasma sanguin par adsorption.

FIG.1

FIG.2

FIG.3

EP 0 420 766 B1

# FIG.4

# FIG.5

# FIG.6